(19) Europäisches Patentamt
European Patent Office
Office européen des brevets

(11) **EP 3 050 958 A1**

(12) **EUROPEAN PATENT APPLICATION**
published in accordance with Art. 153(4) EPC

(43) Date of publication:
**03.08.2016 Bulletin 2016/31**

(51) Int Cl.:
***C12M 1/34*** *(2006.01)*     ***G01N 21/17*** *(2006.01)*

(21) Application number: **13894337.8**

(22) Date of filing: **24.09.2013**

(86) International application number:
**PCT/JP2013/075778**

(87) International publication number:
**WO 2015/045012 (02.04.2015 Gazette 2015/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Fujitsu Limited**
**Kawasaki-shi, Kanagawa 211-8588 (JP)**

(72) Inventors:
 • **MIYAJIMA, Sachiko**
  **Kawasaki-shi**
  **Kanagawa 211-8588 (JP)**
 • **MIYAZAKI, Akira**
  **Kawasaki-shi**
  **Kanagawa 211-8588 (JP)**

 • **SAGA, Susumu**
  **Kawasaki-shi**
  **Kanagawa 211-8588 (JP)**
 • **HAYASHI, Mimiko**
  **Kawasaki-shi**
  **Kanagawa 211-8588 (JP)**
 • **KAWANO, Kiyoshi**
  **Kawasaki-shi**
  **Kanagawa 211-8588 (JP)**
 • **HIDAKA, Kouichi**
  **Kawasaki-shi**
  **Kanagawa 211-8588 (JP)**

(74) Representative: **Hoffmann Eitle**
**Patent- und Rechtsanwälte PartmbB**
**Arabellastraße 30**
**81925 München (DE)**

(54) **COLONY INSPECTION PROGRAM, COLONY INSPECTION DEVICE, AND COLONY INSPECTION METHOD**

(57)     An acquiring unit (111) acquires counted data from each terminal device via an I/F and stores the counted data in a storing unit. A counting unit (112) counts the number of colonies included in a petri dish based on each of petri dish images that are stored as image data. Furthermore, the counting unit (112) calculates an image feature value of each of the petri dish images. A calculating unit (113) calculates, based on the image feature value related to each of the petri dish images stored as image feature value data, the similarity between the associated petri dish images. The judgement unit (114) judges, based on a difference of the number of colonies between the petri dishes and the similarity between the petri dish images, whether a fungus is mixed in the petri dish that is the inspection target. The judgement unit (114) outputs an alarm when the judgement unit judges that a fungus is mixed in the petri dish that is the inspection target.

FIG.2

**Description**

[Technical Field]

The embodiments discussed herein are directed to a colony inspection program, a colony inspection device, and a colony inspection method.

[Background Art]

As an inspection related to food, in order to examine whether a sanitary inspection itself is accurately performed, the inspection quality is sometimes checked other than a sanitary inspection of mixing of fungi. In this inspection quality check, for example, in order to confirm whether there is no problem with the procedure of an inspection, the inspection equipment, and reagents of the inspection that is performed in an inspection room every day, there may be a case of selecting a specimen used for an inspection and performing measurement for each certain period of time or for each certain number of inspections. Furthermore, in a daily sanitary inspection work, there may be a case in which the inspection quality is confirmed. There is a device that automatically counts the number of colonies from an image of a petri dish that is captured.

[Citation List]

[Patent Citation]

Patent Document 1: Japanese Laid-open Patent Publication No. 2011-239683
Patent Document 2: Japanese Laid-open Patent Publication No. 2011-212013

[Summary of Invention]

[Technical Problem]

If the device that automatically counts the number of colonies described above is used, other than the sanitary inspection, an inspection of the inspection quality itself can be performed. For example, among a plurality of petri dishes that are created from the same specimen under the same condition, the number of colonies is supposed to be equal. Thus, if a difference between the number of colonies among the plurality of petri dishes that are treated in the sanitary inspection and that are created from the same specimen under the same condition is within a certain range, it can be determined that there is no problem with the quality of the sanitary inspection. However, with the technology described above, there may be a case in which the inspection quality is not able to appropriately be evaluated.
Namely, in some cases, just comparing the number of colonies in the plurality of petri dishes by using the colony automatic counting device may be insufficient for appropriately evaluating the inspection quality. The reason is that, in some cases, an accident (contamination) in the process of culture or deficiencies of inspection environment is not able to be detected only from the information about the number of colonies. For example, even if the number of colonies is equal among the plurality of petri dishes, if one of the petri dishes is contaminated due to a touch of a finger of an inspector in the process of culture, the type or the size of fungi developed in the respective petri dishes may possibly differ. In such a case, if the number of colonies among the plurality of petri dishes is equal, in a check of the inspection quality based on the comparison of the number of colonies, in some cases, it may be determined that there is no problem with the quality of the sanitary inspection.
Accordingly, it is an object in one aspect of an embodiment of the invention to provide a colony inspection program, a colony inspection device, and a colony inspection method that can appropriately evaluate inspection quality.

[Solution to Problem]

According to a first aspect, a colony inspection program causes a computer to execute a process including: acquiring each of captured images of a plurality of petri dishes in each of which bacterial colonies are included; and calculating similarity between the plurality of the images. Furthermore, the colony inspection program causes the computer to execute a process including controlling, based on the calculated similarity, whether an alarm is output.

[Advantageous Effects of Invention]

The quality of sanitary inspection can be appropriately evaluated.

[Brief Description of Drawings]

FIG. 1 is a schematic diagram illustrating the configuration of the entire system according to a first embodiment.
FIG. 2 is a functional block diagram illustrating the configuration of a colony inspection device according to the first embodiment.
FIG. 3 is a schematic diagram illustrating an example of the data structure of an inspection DB.
FIG. 4 is a schematic diagram illustrating a first example of a color histogram.
FIG. 5 is a schematic diagram illustrating a second example of the color histogram.
FIG. 6 is a schematic diagram illustrating a first example of a distribution histogram of colony areas.
FIG. 7 is a schematic diagram illustrating a second example of the distribution histogram of the colony areas.
FIG. 8 is a schematic diagram illustrating a first example of an alarm display on a judgement screen.
FIG. 9 is a schematic diagram illustrating a second example of the alarm display on the judgement screen.
FIG. 10 is a schematic diagram illustrating an example of a processing matrix of the colony inspection device.
FIG. 11 is a schematic diagram illustrating a first example of a processing operation performed until the colony inspection device outputs an alarm.
FIG. 12 is a schematic diagram illustrating a second example of the processing operation performed until the colony inspection device outputs the alarm.
FIG. 13 is a schematic diagram illustrating a third example of the processing operation performed until the colony inspection device outputs the alarm.
FIG. 14 is a block diagram illustrating an example of the hardware configuration of a computer according to the colony inspection device.

[Embodiments for Carrying Out the Invention]

Preferred embodiments of a colony inspection program, a colony inspection device, and a colony inspection method disclosed in the present invention will be described in detail below with reference to the accompanying drawings. The present invention is not limited to the embodiments. The embodiments can be appropriately used in combination as long as processes do not conflict with each other.

First Embodiment

(Configuration of the entire system)

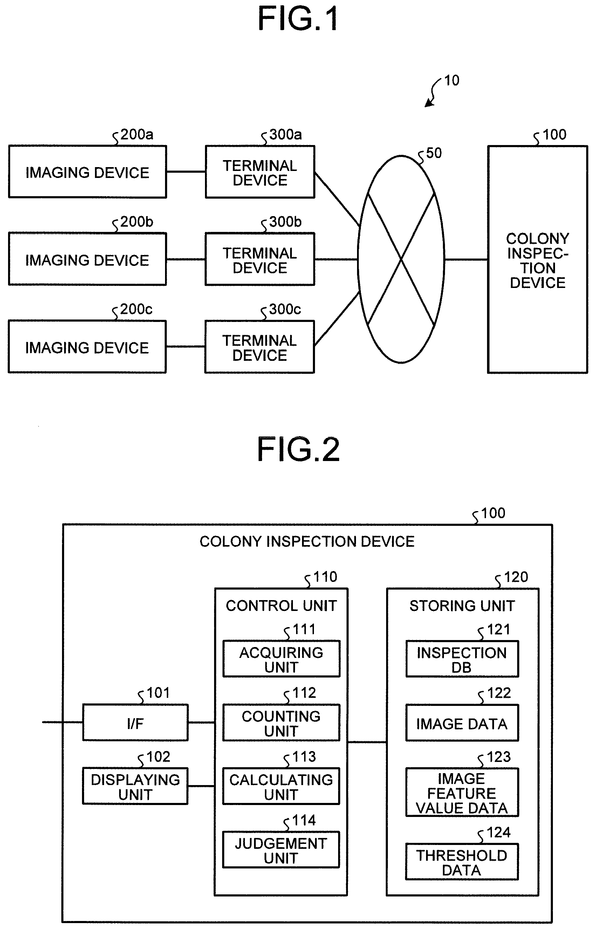

In the following, the configuration of the entire system according to a first embodiment will be described. FIG. 1 is a schematic diagram illustrating the configuration of the entire system according to a first embodiment. As illustrated in FIG. 1, a system 10 includes imaging devices 200a to 200c, terminal devices 300a to 300c, a network 50, and a colony inspection device 100.

The imaging device 200a is connected to the terminal device 300a. Furthermore, the imaging device 200b is connected to the terminal device 300b. Furthermore, the imaging device 200c is connected to the terminal device 300c. Hereinafter, an image of a petri dish captured by each of the imaging devices is referred to as a petri dish image. Each of imaging devices 200 captures an image of a placed petri dish and sends image data to the associated terminal devices 300. The terminal devices 300 send the image data to the colony inspection device 100 via the network 50. The colony inspection device 100 judges, based on the image data sent from each of the terminal devices 300, whether the petri dish was imperfect in the process of inspection. If the colony inspection device 100 judges that there is a high possibility that the petri dish was imperfect, the colony inspection device 100 outputs an alarm.

Furthermore, in the first embodiment, an example in which each of the imaging devices 200 captures a petri dish image and the colony inspection device 100 counts the number of colonies from the petri dish image is exemplified; however, each of the terminal devices 300 may also count the number of colonies based on the petri dish image. Furthermore, a description of the process in which the colony inspection device 100 outputs an alarm will be given in detail later.

(Creation of a petri dish in an inspection department)

In the following, the creation of a petri dish that is placed in each of the imaging devices 200 will be described. The inspection department is a department that performs sanitary inspection in a food manufacturing plant. An inspector who belongs to the inspection department conducts a sanitary inspection. In contrast, a responsible person who belongs to the same inspection department performs final judgement of the sanitary inspection performed by the inspector and evaluates the quality of the sanitary inspection performed by the inspector.

In order to perform the sanitary inspection of food to be shipped, the inspector in the inspection department collects specimens from the food and creates a petri dish by a predetermined method.

For example, the inspector creates a petri dish as follows. First, the inspector attaches an inspection No, which is an identification number and is used to distinguish from other specimens, to the specimens collected from food that is the inspection target. Then, the inspector writes, on the lid of each of two petri dishes, the same number as that of the inspection No attached to the specimens. Then, the inspector grinds the specimens and adds dilution water, whereby the inspector creates a sample undiluted solution. The inspector extracts some of the sample undiluted solution by a dropper, adds the extracted sample undiluted solution to the two petri dishes, dilutes to a predetermined concentration by further adding dilution water, and creates two petri dishes with a first dilution step. Then, the inspector pours culture media, such as agar or the like, into the petri dishes and pours the culture media in the petri dishes.

Then, after the agar became solidified, the inspector turns the petri dishes upside down with the lid of each of the petri dishes facing down, keeps the temperature inside the two petri dishes at a predetermined temperature by using an incubator, leaves each of the petri dishes for about one or two days. Consequently, fungi are cultured in the two petri dishes from the same specimen under the same condition. Some of the specimens collected from the food are cryopreserved for reexamination. Subsequently, the inspector places the created petri dishes in each of the imaging devices 200. Furthermore, in the above description, two petri dishes with the same dilution step are created from the beginning; however, it may also possible to create two petri dishes with consecutive the second and the third steps, select one of the appropriate dilution step, and count the number of colonies.

(About a use of the colony automatic counting device)

Furthermore, in general, a count work of the number of colonies is visually performed. However, in order for the inspector to accurately perform the count work, the inspector needs a certain level of work experience. Thus, the inspection department is not able to request an inspector with insufficient work experience to perform the count work. Furthermore, because the inspection department needs to steadily perform tasks with a limited number of inspectors, there is a circumstance that it is difficult for an inspector who has an inspection work experience inform another inspector of the know-how of the count work. Furthermore, because an error occurs in a count due to a difference between the work experiences of each of the inspectors, there is a problem in that it is difficult for the inspection department to keep the inspection level of a count work constant. Because of these circumstances, if the inspection department allows an inspector to visually perform a count work, it is difficult to keep the quality of a work constant.

Thus, an inspector increasingly uses, for an inspection, a colony automatic counting device that automatically counts the number of colonies in a petri dish. Because the colony automatic counting device automatically counts the number of colonies, the device may also be used even for a less work experienced inspector. Furthermore, by allowing an inspector to use the colony automatic counting device, the inspection department can easily keep the inspection level constant. Consequently, the inspection department increasingly allows an inspector to use the colony automatic counting device.

(Functional configuration of the colony inspection device)

An example of the functional configuration of the colony inspection device 100 according to the first embodiment will be described. FIG. 2 is a functional block diagram illustrating the configuration of a colony inspection device according to the first embodiment. As illustrated in FIG. 2, the colony inspection device 100 includes an interface (I/F) 101, a displaying unit 102, a control unit 110, and a storing unit 120. The I/F 101 is a communication interface that is connected to the network 50 and that sends data to each of the terminal devices 300 via the network 50. The displaying unit 102 displays the processing result obtained from the colony inspection device 100 on a monitor. Furthermore, the displaying unit 102 displays an alarm output from the colony inspection device 100.

(Each configuration of the storing unit)

The storing unit 120 stores therein an inspection database (DB) 121, image data 122, image feature value data 123, and threshold data 124. The storing unit 120 corresponds to, for example, semiconductor memory device, such as a random access memory (RAM), a read only memory (ROM), a flash memory, and the like, or a storage device, such as a hard disk, an optical disk, and the like.

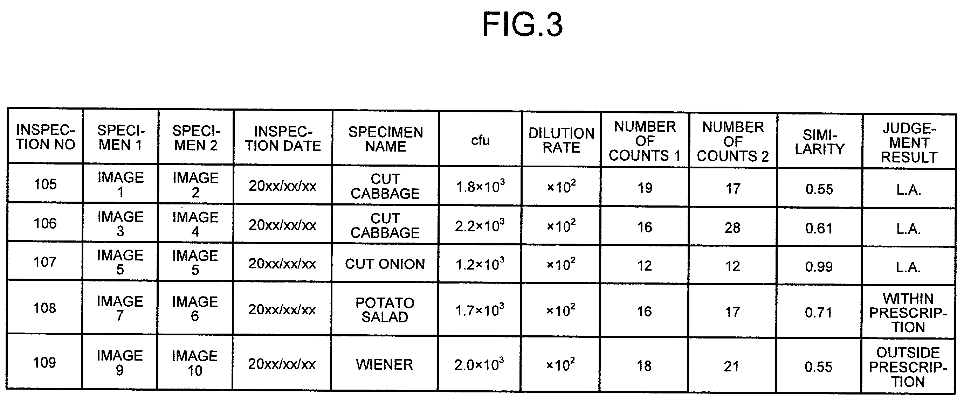

The inspection DB 121 is a database that stores therein, in an associated manner for each inspection, data related to an inspection, the similarity between the petri dish images, and the judgement result. FIG. 3 is a schematic diagram illustrating an example of the data structure of an inspection DB. As illustrated in FIG. 3, the inspection DB 121 associates an inspection No, a specimen 1, a specimen 2, the inspection date, the specimen name, cfu, the dilution rate, the number of counts 1, the number of counts 2, the similarity, and the judgement result. The "inspection No" is a number that is uniquely attached to each specimen on the inspection date. Furthermore, a single "inspection No" is attached to a specimen group created from the same petri dish. The "specimen 1" indicates the image of the specimen 1 that is one of the two specimens created from the same petri dish. The "specimen 2" indicates the image of the specimen 2 that is the other one of the two specimens created from the same petri dish. The "inspection date" indicates the date on which an inspection is performed. The "specimen name" indicates the name of food from which specimens are collected. The "cfu" indicates the number of fungi in a specimen per gram. The "dilution rate" indicates the rate of dilution of the original solution. The "number of counts 1" indicates the number of colonies included in the specimen 1. The "number of counts 2" indicates the number of colonies included in the specimen 2. The "similarity" indicates the similarity between images related to the specimen 1 and the specimen 2. The "similarity" is indicated by 0 to 1 and, if the images to be compared are the same, the "similarity" becomes 1. The "judgement result" indicates the result judged by a responsible person. For example, if a responsible person determines that the cfu counted by inspector is within a prescribed value that is defined for each food group, the "judgement result" becomes "within the prescription". Furthermore, if the responsible person determines that the cfu exceeds the prescribed value that is defined for each food group, the "judgement result" becomes "outside the prescription". Furthermore, if the responsible person determines that there is a problem in the inspection quality, the "judgement result" becomes an "Laboratory Accident (L.A.)".

The image data 122 is data on a petri dish image received from each of the terminal devices 300. As will be described later, the colony inspection device 100 associates, by using the inspection DB 121, image data related to the two petri dishes created by dividing an inspection target specimen and stores the associated image data as the image data 122. The file type of the image data 122 is, for example, a GIF file, a JPEG file, a BMP file, or the like.

The image feature value data 123 is data related to an image feature value of each of the associated petri dish images. The colony inspection device 100 acquires the image feature value data 123 by using various kinds of methods. For example, the colony inspection device 100 creates a color histogram associated with each of the petri dish images. Then, the colony inspection device 100 stores the created color histogram in the storing unit 120 as the image feature value data 123. Furthermore, the colony inspection device 100 calculates, based on the petri dish image, an average value of the colony area. Then, the colony inspection device 100 stores the average value of the colony area in the storing unit 120 as the image feature value data 123. Furthermore, the colony inspection device 100 calculates a Scale-Invariant Feature Transform (SIFT) feature value of each of the petri dish images. Then, the colony inspection device 100 stores the SIFT feature values in the storing unit 120 as the image feature value data 123. Furthermore, the colony inspection device 100 may also calculate an image feature value by using the Speeded Up Robust Features (SURF) or the histogram of oriented gradients (HOG). The above description is an example of an image feature value calculated by the colony inspection device 100 and the colony inspection device 100 may also calculate an image feature value by using another method. Furthermore, calculation of an image feature value will be described in detail later.

The threshold data 124 is data indicating each threshold that is used when the colony inspection device 100 judges whether an alarm is output. As will be described later, the colony inspection device 100 calculates the similarity between the associated petri dish images and judges whether an alarm is output in accordance with whether the similarity is equal to or greater than the threshold. For example, if the colony inspection device 100 compares petri dish images of vegetables, the colony inspection device 100 sets the threshold to "0.6" based on the threshold data 124. Furthermore, the colony inspection device 100 may also appropriately update the threshold based on a feedback from the responsible person. Furthermore, an update of the threshold will be described in detail later.

(Each configuration of the control unit)

The control unit 110 includes an acquiring unit 111, a counting unit 112, a calculating unit 113, and a judgement unit 114. The function of the control unit 110 can be implemented by, for example, a CPU (central processing unit) executing a predetermined program. Furthermore, the function of the control unit 110 can be implemented by, for example, an integrated circuit, such as an application specific integrated circuit (ASIC), a field programmable gate array (FPGA), or the like. Furthermore, the judgement unit 114 is an example of an output unit.

The acquiring unit 111 acquires count data from each of the terminal devices 300 via the I/F 101 and stores the count data in the storing unit 120. For example, the acquiring unit 111 receives, from each of the terminal devices 300, two petri dish images created by dividing a specimen that is the inspection target. Then, the acquiring unit 111 associates the associated two petri dish images with a single inspection No and stores the associated two petri dish images in the inspection DB 121. In addition to this, the acquiring unit 111 receives, from each of the terminal devices 300 for each inspection, the data on the inspection date, the specimen, the cfu, and the dilution rate, associates the data with the same inspection No, and stores the associated data.

The counting unit 112 counts the number of colonies included in a petri dish based on each of the petri dish images that are stored in the image data 122. Furthermore, the counting unit 112 calculates an image feature value of each of the petri dish images. For example, the counting unit 112 counts each of the number of colonies included in a petri dish based on the petri dish image received from each of the terminal devices 300. Then, if the image of one of the petri dishes created from the same specimen is defined as an image 1 and the image of the other one is defined as an image 2, the counting unit 112 enters the number of colonies of the same image 1 into the "number of counts 1" in the inspection DB 121 and enters the number of colonies of the image 2 into the "number of counts 2". Furthermore, the colonies in the petri dish overlap, the counting unit 112 may also count the colonies by separating the colonies by using image processing.

Then, the counting unit 112 calculates, as image feature values of the image 1 and the image 2, a color histogram, the average value of the areas of colonies, a SIFT feature value, or the like. For example, when the counting unit 112 obtains a color histogram as the image feature value of each of the image 1 and the image 2, the counting unit 112 processes as follows. First, the counting unit 112 reduces the number of each of the color components of the red component R, the green component G, and the blue component B in each of the pixels included in the image 1 and the image 2 to four colors and sets the number of combinations of the red component R, the green component G, and the blue component B to 64. Then, the counting unit 112 attaches numbers from 0 to 63 to the colors that are associated with 64 combinations, respectively, and uses the numbers as bin numbers. Then, the counting unit 112 counts the number of pixels that are associated with the bin numbers and that are included in the image 1 and the image 2 and creates a color histogram.

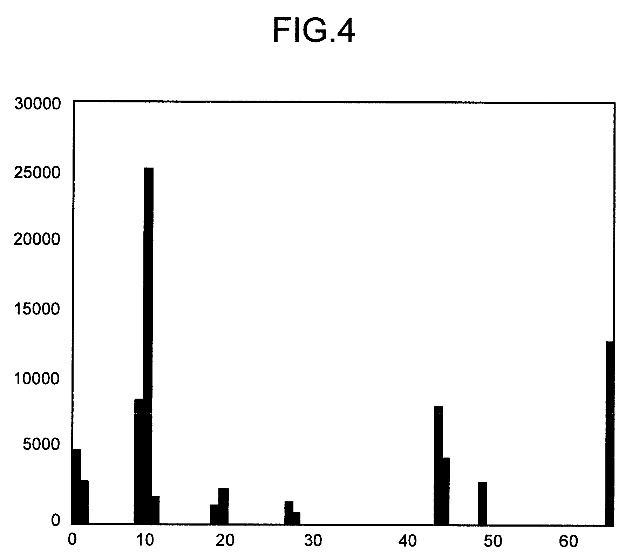

FIG. 4 is a schematic diagram illustrating a first example of a color histogram. As illustrated in FIG. 4, the color histogram represents the number of pixels that are associated with the bin numbers from 0 to 63 in the image 1. For example, the color histogram indicates that the number of pixels with the bin number of 0 is about 5000 in the image 1. Furthermore, the color histogram indicates that about 25000 pixels with the bin number of 10 are present in the image 1. Furthermore, the color histogram indicates that about 1000 pixels with the bin number of 20 are present in the image 1. Furthermore, for the other bin numbers in the image 1, the color histogram also indicates the number of pixels associated with each of the bin numbers.

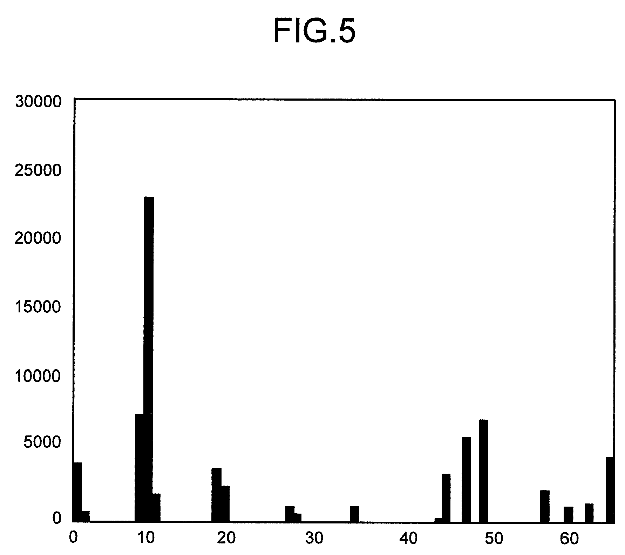

FIG. 5 is a schematic diagram illustrating a second example of the color histogram. As illustrated in FIG. 5, similarly to FIG. 4, the color histogram also represents the number of pixels that are associated with the bin numbers from 0 to 63 in the image 2. For example, the color histogram indicates that about 4000 pixels with the bin number of 0 are present in the image 2. Furthermore, the color histogram indicates that about 23000 pixels with the bin number of 10 are present in the image 2. Furthermore, the color histogram indicates that about 2000 pixels with the bin number of 20 are present in the image 2. Furthermore, for the other bin numbers in the image 2, the color histogram also indicates the number of pixels associated with each of the bin numbers.

Then, the counting unit 112 stores each of the color histogram related to the image 1 illustrated in FIG. 4 and the color histogram related to the image 2 illustrated in FIG. 5 as the image feature value data 123 in the storing unit 120. Calculation of the similarity performed by using a color histogram will be described later.

Furthermore, the histogram created by the counting unit 112 is not limited to a color histogram and another histogram may also be created. For example, the counting unit 112 may also create a distribution histogram of a colony area in the following procedure. First, the counting unit 112 performs edge extraction process on the image 1 and detects a closed portion as a colony. Then, the counting unit 112 calculates the area of each of the colonies included in the image 1. Similarly, the counting unit 112 calculates the area of each of the colonies included in the image 2.

Then, the counting unit 112 classifies the colonies in the image 1 into N levels in accordance with the size of the areas and counts the number of colonies. For example, if the counting unit 112 classifies the areas of the colony into seven levels, the counting unit 112 counts the number of colonies belonging to the region with the smallest area in the first level. Furthermore, the counting unit 112 counts the number of colonies belonging to the region with the second smallest area in the second level. In this way, the counting unit 112 counts the number of colonies belonging to each of the levels. Then, the counting unit 112 represents the number of colonies on the vertical axis and represents the areas of colonies on the horizontal axis and creates a distribution histogram of the colony areas in the image 1. Furthermore, similarly, the counting unit 112 creates a distribution histogram of the colony areas in the image 2.

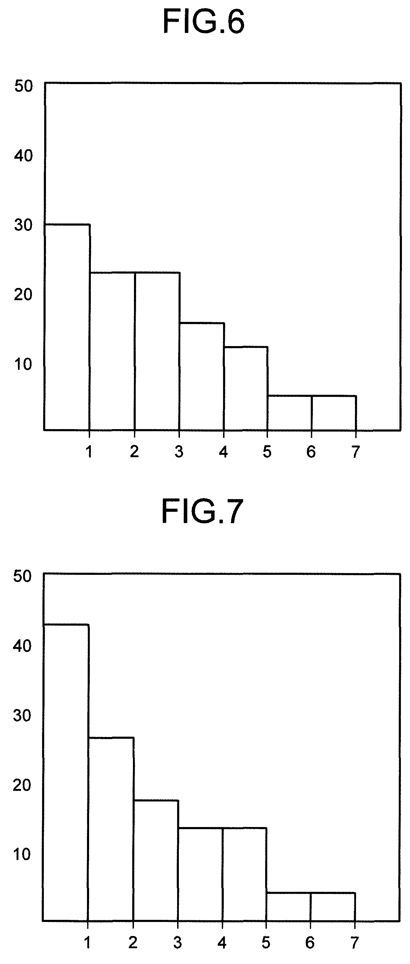

FIG. 6 is a schematic diagram illustrating a first example of a distribution histogram of colony areas. The distribution histogram of the colony areas illustrated in FIG. 6 is associated with the image 1. Furthermore, the distribution histogram of the colony areas represents the number of colonies in the vertical axis direction and represents the colony areas in the horizontal axis direction. Furthermore, the distribution histogram of the colony areas classifies the colony areas into seven levels and indicates the number of colonies at each of the levels. For example, in contrast, the distribution histogram of the colony areas indicates that the number of colonies at the first level is about 30. Furthermore, the distribution histogram of the colony areas indicates that the number of colonies at the second level is about 23. The distribution histogram of the colony areas indicates the number of colonies at each of the other levels. In contrast, FIG. 7 is a schematic diagram illustrating a second example of the distribution histogram of the colony areas. The distribution histogram of the colony areas illustrated in FIG. 7 is associated with the image 2. Furthermore, calculation of the similarity performed by using the distribution histogram of the colony areas will be described later.

Furthermore, the counting unit 112 may also calculate an image feature value by using another method. For example, the counting unit 112 may also calculate the average value of the colony areas as the image feature value data. Specifically, the counting unit 112 performs the edge extraction process on the image 1 and the image 2 and detects a closed portion as a colony. Furthermore, the counting unit 112 may also detect a colony by using the area of a closed portion, the color, the size, or the like. Then, the counting unit 112 obtains the total area of a colony by counting the number of pixels of each of the detected colonies. Then, the counting unit 112 obtains the average value of the areas of the colonies by dividing the total area of the colonies by the number of colonies. Then, the counting unit 112 stores the average value of the areas of the colonies as the image feature value data 123 in the storing unit 120.

Alternatively, the counting unit 112 may also calculate a SIFT feature value of each of the image 1 and the image 2 and allow stores the storing unit 120 to store the calculated value as the image feature value data 123. Furthermore, the SIFT is an algorithm used for object recognition and is said to be strong in rotation or scaling of an image and strong in illumination change. The counting unit 112 obtains a SIFT feature value by detecting a feature point from an image and calculating a brightness change for each feature point. Then, the counting unit 112 allows the storing unit 120 to store each of the obtained SIFT feature values as the image feature value data 123.

The calculating unit 113 calculates the similarity between the associated petri dish images based on the image feature value related to each of the petri dish images stored in the image feature value data 123. For example, if the counting unit 112 calculates the color histograms of the image 1 and the image 2 as the image feature values, the calculating unit 113 compares the histograms in the following procedure by using a method called Histogram Intersection. The value of each of the bin numbers indicates the number of pixels corresponding to each of the bin numbers. First, the calculating unit 113 compares the values of the bin number 0 of the color histograms of the image 1 and the image 2 and then adds a smaller value to the total value. For example, if it is assumed the value of the bin number 0 of the image 1 is "5000" and the value of the bin number 0 of the image 2 is "4000", the calculating unit 113 sets the total value to "4000" by adding the smaller value of "4000" to the total value of "0". Then, if it is assumed that the value of the bin number 1 of the image 1 is "2000" and the value of the bin number 1 of the image 2 is "1500", the calculating unit 113 sets the total value to "5500" by adding the smaller value of "1500" to the total value of "4000". Then, if it is assumed that the value of the bin number 2 of the image 1 is "1000" and the value of the bin number 2 of the image 2 is "1500", the calculating unit 113 sets the total value to "6500" by adding the smaller value of "1000" to the total value of "5500". Furthermore, similarly to the bin numbers 3 to 63, the calculating unit 113 adds the smaller value to the total value and calculates the total value. Furthermore, similarly to the image 2, the calculating unit 113 calculates a total value X1. Furthermore, the total value X1 calculated by the calculating unit 113 can be represented by the following Equation, where the value of the bin number i of the image 1 is set to H1 [i] and the value of the bin number i of the image 2 is set to H2 [i].

$$X_1 = \sum_{i=0}^{63} \min\left(H_1[i], H_2[i]\right) \qquad (1)$$

Then, the calculating unit 113 calculates a similarity X2 indicated by the range from 0 to 1 by dividing the obtained total value by the total number of pixels of the image 1 or the image 2. Namely, the similarity X2 can be represented by the following Equation, where the value of the bin number i of the image 1 is H1 [i] and the value of the bin number i of the image 2 is H2 [i].

$$X_2 = \frac{\sum_{i=0}^{63} \min\left(H_1[i], H_2[i]\right)}{\sum_{i=0}^{63} H_1[i]} \qquad (2)$$

Furthermore, the method of comparing the histograms using Histogram Intersection described above is an example and the calculating unit 113 may also compare the histograms by using another method. For example, the calculating unit 113 may also compare histograms by using the Bhattacharyya distance. First, the counting unit 112 performs normalization by dividing the value of each of the bin numbers of the histogram of the image 1 by the total number of pixels of the image 1. Consequently, the histogram of the image 1 is represented by the probability of occurrence distribution. Then, by using the same procedure, the counting unit 112 represents the histogram of the image 2 by the probability of occurrence distribution.

Then, for each of the bin numbers, the calculating unit 113 sets the sum of the square root of the product of the probability of occurrence in the image 1 and the image 2 as the similarity X3 of the image 1 and the image 2. Consequently, the similarity takes the value from 0 to 1. Namely, in this case, the similarity X3 can be represented by the following equation, where the probability of occurrence of the bin number i of the image 1 is p [i] and the probability of occurrence of the bin number i of the image 2 is q [i].

$$X_3 = \sum_{i=0}^{63} \left\{ \sqrt{p[i] \cdot q[i]} \right\} \qquad (3)$$

Furthermore, if the counting unit 112 calculates the histogram of the colony area of each of the image 1 and the image 2 as an image feature value, the calculating unit 113 may also calculate the similarity by using the following procedure. First, the calculating unit 113 compares the values at the first level, in the histogram, of the colony areas of the image 1 and the image 2 and adds the smaller value to the total value. Then, the calculating unit 113 compares the values at the second level, in the histogram, of the colony areas of the image 1 and the image 2 and adds the smaller value to the total value. The calculating unit 113 calculates the total value by repeatedly performing the calculation described above up to the seventh level that is the largest region of the colony area. Then, the calculating unit 113 calculates the similarity X4 of the image 1 and the image 2 by dividing the calculated total value by the total number of colonies of the image 1 or the image 2. Namely, in this case, the similarity X4 can be represented by the following equation, where the number of colonies at the $i^{th}$ level of the image 1 is H1 [i] and the number of colonies at the $i^{th}$ level of the image 2 is H2 [i].

$$X_4 = \frac{\sum_{i=0}^{N} \min(H_1[i], H_2[i])}{\sum_{i=0}^{N} H_1[i]} \qquad (4)$$

Furthermore, the calculating unit 113 may also calculate the similarity by using the average value of the areas of each of the colonies of the image 1 and the image 2. For example, the calculating unit 113 calculates the similarity by dividing the image 1 in which the average value of the colony areas is small by the image 2 in which the average value of the colony areas is large. It is assumed that, if the same type of colonies is present in the two petri dishes, the similarity becomes closer to 1.

Furthermore, the calculating unit 113 may also calculate the similarity by using the SIFT feature value of the image 1 and the image 2. For example, the calculating unit 113 creates a feature vector from the SIFT feature value of each of the image 1 and the image 2 and calculates the similarity based on each of the feature vectors.

The judgement unit 114 judges whether the inspection quality is low based on the difference of the number of colonies between the petri dishes and the similarity between the petri dish images. If the judgement unit 114 judges that the inspection quality is low, the judgement unit 114 outputs an alarm. For example, if the difference of the number of colonies between the petri dishes is equal to or greater than a threshold, the judgement unit 114 judges that the petri dish of the inspection target was imperfect in the process of inspection and outputs an alarm. Furthermore, if the similarity between the petri dish images is equal to or less than the threshold, the judgement unit 114 judges that the petri dish that is the inspection target was imperfect in the process of the inspection and outputs the alarm. Specifically, if the judgement unit 114 judges that the petri dish that is the inspection target was imperfect in the process of the inspection, the judgement unit 114 displays the alarm indicating that there is a high possibility that the petri dish was imperfect in the process of the inspection on the screen on which a responsible person judges the inspection result. Furthermore, the threshold is set for each food group of the inspection item. Furthermore, displaying on the judgement screen in detail will be described later.

The presence or absence of an output of the alarm performed by the judgement unit 114 will be described with reference of FIG. 3. For example, it is assumed that the judgement unit 114 outputs the alarm when the counts of the number of colonies in the petri dishes differ by "7" or more or when the similarity between the petri dish images is equal to or less than "0.6". In this case, for the inspection No "105" in the inspection DB 121 illustrated in FIG. 3, because the similarity is "0.55" and thus the similarity is equal to or less than "0.6", the judgement unit 114 judges that the condition of the alarm is satisfied and thus outputs the alarm to a judgement screen 400. Furthermore, for the inspection No "106", because the difference between the number of counts 1 and the number of counts 2 is "12" and thus the difference is equal to or greater than "7", the judgement unit 114 judges that the condition of the alarm is satisfied and thus outputs the alarm to the judgement screen 400. Furthermore, for the inspection No "108", because the difference between the number of counts 1 and the number of counts 2 is "1", i.e., the number of counts is smaller than "7", and because the similarity is ""0.71", i.e., the similarity is greater than "0.6", the judgement unit 114 judges that the condition of the alarm is not satisfied and thus does not output the alarm to the judgement screen 400. Furthermore, for the inspection No "109", because the similarity is "0.55", i.e., the similarity is equal to or less than "0.6", the judgement unit 114 judges that the condition of the alarm is satisfied and thus outputs the alarm to the judgement screen 400. Furthermore, an output of the alarm when the inspection No is "107" will be described later.

Furthermore, the judgement unit 114 may also judge whether or not to output the alarm in accordance with the count ratio of the number of colonies between the petri dishes. For example, if the count ratio of the number of colonies between the petri dishes is more than a factor of four or if the similarity between the petri dish images is equal to or less than "0.6", the judgement unit 114 may also output the alarm. In this case, if the number of counts 1 is "4" and the number of counts 2 is "17", because the number of counts 2 is equal to or greater than four times the number of counts 1, the judgement unit 114 judges that the alarm condition is satisfied and then outputs the alarm to the judgement screen 400. In contrast, if the number of counts 1 is "4" and the number of counts 2 is "15" and if the similarity between the petri dish images is equal to or greater than "0.6", the judgement unit 114 judges that the alarm condition is not satisfied and does not output an alarm to the judgement screen 400.

(Example of an alarm display on a judgement screen)

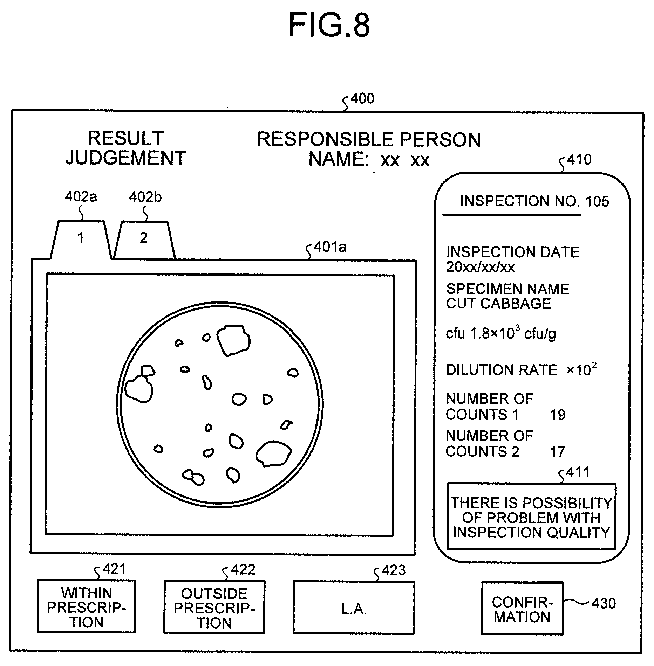

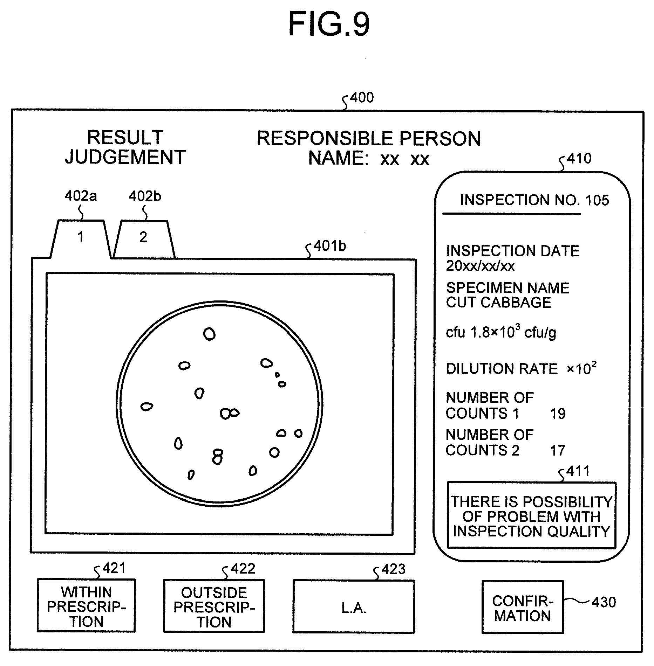

FIG. 8 is a schematic diagram illustrating a first example of an alarm display on a judgement screen. Furthermore, FIG. 9 is a schematic diagram illustrating a second example of the alarm display on the judgement screen. FIG. 8 is associated with the display of the image 1 related to the inspection No "105" illustrated in FIG. 3. Furthermore, FIG. 9 is associated with the display of the image 2 related to the inspection No "105" illustrated in FIG. 3. The judgement screen 400 displays, for example, when a tab 402a is selected, as illustrated in FIG. 8, the petri dish image related to the image 1 on an image displaying unit 401a. In contrast, the judgement screen 400 displays, when a tab 402b is selected, as illustrated in FIG. 9, the petri dish image related to the image 2 on an image displaying unit 401b. Furthermore, the displaying unit 102 displays, on an inspection information display list 410 on the judgement screen 400, the "inspection No", the "inspection date", the "specimen name", the "cfu", the "dilution rate", the "number of counts 1", and the "number of counts 2". Furthermore, if the judgement unit 114 determines that there is a high possibility of imperfection in the process of the inspection, the displaying unit 102 displays, as illustrated in FIG. 8 and FIG. 9, on an alarm displaying unit 411 in the inspection information display list 410, an alarm indicating that "there is a possibility of a problem with the inspection quality".

Furthermore, the displaying unit 102 displays, on the lower portion of the judgement screen 400, a within-the-prescription button 421, an outside-the-prescription button 422, an L.A. button 423, and a confirmation button 430. When the within-the-prescription button 421 is pressed by a responsible person in the inspection department and the confirmation button 430 is further pressed, the colony inspection device 100 allows the inspection DB 121 to store the judgement result indicating "within the prescription". Furthermore, when the outside-the-prescription button 422 is pressed by the responsible person in the inspection department and the confirmation button 430 is further pressed, the colony inspection device 100 allows the inspection DB 121 to store the judgement result indicating "outside the prescription". Furthermore, the L.A. button 423 is pressed by the responsible person in the inspection department and the confirmation button 430 is further pressed, the colony inspection device 100 allows the inspection DB 121 to store the judgement result indicating "L.A.".

Furthermore, if the judgement result is "within the prescription", the inspection department determines that the number of colonies in the inspected specimen is within the defined prescribed value. Furthermore, is the judgement result is "outside the prescription", the inspection department determines that the number of colonies in the inspected specimen exceeds the defined prescribed value and immediately reports an inspection request source, such as a manufacturing plant line or the like. Furthermore, if the judgement result is "L.A.", the inspection department determines that an inspection is again needed and notifies the inspector of an instruction to again create a petri dish by using the same specimen that is cryopreserved. Furthermore, the inspection department guides the inspector in order to improve the inspection work.

In this way, if there is a high possibility of imperfection in the process of the inspection, the colony inspection device 100 can provide useful information, by outputting the alarm to the judgement screen 400, for judging whether there is a problem with the inspection quality. Furthermore, instead of outputting the alarm to the judgement screen 400, the displaying unit 102 may also output the alarm by displaying a pop up. Furthermore, the colony inspection device 100 may also output the alarm by sending a mail to the responsible person.

(Processing matrix of the colony inspection device)

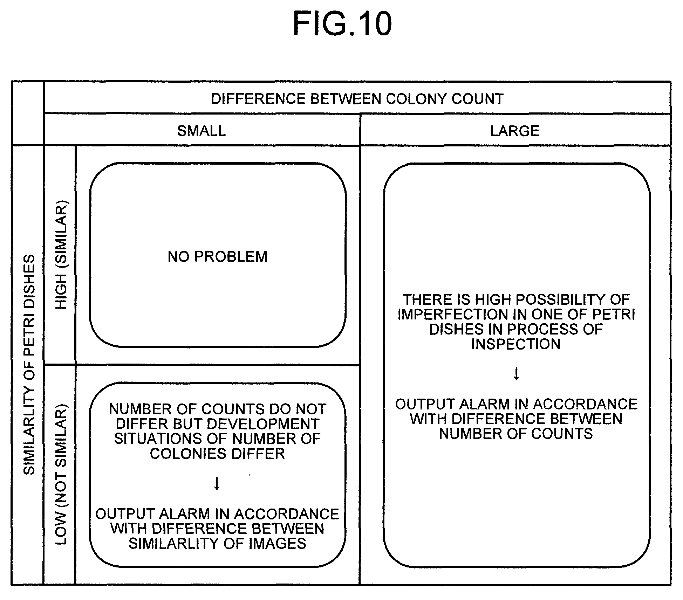

In the following, the content of the process performed by the colony inspection device 100 by using the processing matrix illustrated in FIG. 10 will be described. FIG. 10 is a schematic diagram illustrating an example of a processing matrix of the colony inspection device. If the difference of the number of colonies between the petri dishes counted by the counting unit 112 is equal to or greater than a threshold, the judgement unit 114 judges that there is a high possibility of imperfection in one of the petri dishes in the process of the inspection. In this case, the displaying unit 102 outputs the alarm to the judgement screen 400.

Furthermore, if the difference of the number of colonies between the petri dishes is within the threshold and the similarity between the petri dish images is equal to or greater than the threshold, the judgement unit 114 judges that there is no problem with the inspection quality. In this case, the displaying unit 102 does not output the alarm to the judgement screen 400. Furthermore, if the difference of the number of colonies between the petri dishes is within the threshold and the similarity between the petri dish images is equal to or less than the threshold, because a development situation of the number of colonies is estimated to differ, the judgement unit 114 is judges that there is a high possibility of imperfection in one of the petri dishes in the process of the inspection. In this case, the displaying unit 102 outputs the alarm to the judgement screen 400.

(Flow of the process performed by the colony inspection device)

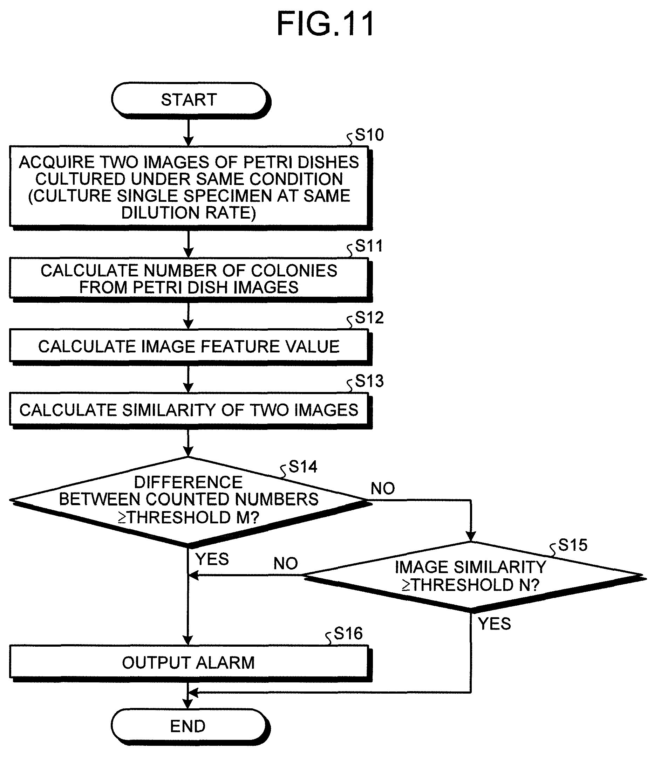

In the following, the flow of the process performed by the colony inspection device 100 will be described. FIG. 11 is a schematic diagram illustrating a first example of a processing operation performed until the colony inspection device outputs the alarm. First, after the acquiring unit 111 divides a single specimen into two petri dishes at the same dilution rate, the acquiring unit 111 acquires each of the images related to the two petri dishes that are created by being cultured in the same environment (Step S10). The acquiring unit 111 associates the associated two petri dish images with a single inspection No and allows the inspection DB 121 to store the associated information in.

Then, the counting unit 112 calculates the number of colonies from each of the associated petri dish images that are stored in the inspection DB 121 (Step S11). Then, the counting unit 112 calculates an image feature value of each of the associated two petri dish images (Step S12). For example, the counting unit 112 calculates a color histogram of each of the two petri dish images as the image feature values. The counting unit 112 allows the storing unit 120 to store the calculated image feature value in the image feature value data 123.

Then, the calculating unit 113 calculates the similarity of the two petri dish images (Step S13). For example, the calculating unit 113 compares the values of the respective bin numbers that are in the color histogram and that are associated with the two petri dish images and obtains a total value by summing a smaller value of each of the bin numbers. Then, the calculating unit 113 calculates the similarity by dividing the obtained total value by one of the total number of pixels of the two petri dish images.

Then, if the difference of the number of colonies between the petri dishes counted by the counting unit 112 is equal to or greater than a threshold M (Yes at Step S14), the judgement unit 114 outputs the alarm (Step S16) and ends the process. In contrast, if the difference of the number of colonies between the petri dishes counted by the counting unit 112 is smaller than the threshold M (No at Step S14), the judgement unit 114 moves to the process at Step S15.

Then, if the image similarity is smaller than the threshold N at Step S15 (No at Step S15), the judgement unit 114 outputs the alarm (Step S16) and ends the process. In contrast, if the image similarity is equal to or greater than the threshold N at Step S15 (Yes at Step S15), the judgement unit 114 does not output the alarm and ends the process.

(Effects)

Namely, the colony inspection device 100 includes the acquiring unit 111 that acquires each of captured images of a plurality of petri dishes in each of which bacterial colonies are included and the calculating unit 113 that calculates the similarity between the plurality of the images. Furthermore, the colony inspection device 100 includes, based on the calculated similarity, the judgement unit 114 that changes whether the alarm is output.

Furthermore, if the calculated similarity indicates a difference equal to or greater than the predetermined value, the judgement unit 114 outputs the alarm.

Furthermore, the colony inspection device 100 may also include the counting unit 112 that counts the number of bacterial colonies included in each of the plurality of images. Then, if a difference between the values counted for each of the plurality of images is equal to or greater than a predetermined value and if the similarity indicates a difference equal to or greater than the predetermined value, the judgement unit 114 may also output the alarm.

Consequently, the colony inspection device 100 can appropriately evaluate the inspection quality by taking into consideration an element other than the difference of the number of colonies of the two associated petri dish images.

(Another embodiment related to the first embodiment)

In the first embodiment, the colony inspection device 100 calculates the number of colonies of each of the petri dishes and calculates an image feature value of each of the petri dish images; however, the embodiment is not limited to this. Each of the terminal devices 300 illustrated in FIG. 1 may also calculate, based on each of the petri dish images captured by the imaging device 200, the number of colonies in each of the petri dish and one or both the image feature value of each of the petri dish images and send the calculated data to the colony inspection device 100 and then the colony inspection device 100 may also use the data.

In the first embodiment, if the judgement unit 114 judges that there is a high possibility of imperfection in the process of the inspection of the petri dish, the displaying unit 102 outputs the alarm on the judgement screen 400; however, the embodiment is not limited to this. The judgement unit 114 may also output the alarm by displaying a pop-up in addition to the judgement screen 400. Furthermore, the judgement unit 114 may also output the alarm by sending a mail to the responsible person or the like.

In the first embodiment, it has been described that the counting unit 112 calculates a SIFT feature value; however, the embodiment is not limited to this. The counting unit 112 may also calculate an image feature value by using the Speeded Up Robust Features (SURF) or the Histogram of Oriented Gradients (HOG).

In the first embodiment, it has been described that the counting unit 112 calculates a color histogram, a histogram of a colony area, the average value of the area of the colonies, or a SIFT feature value as an image feature value of a petri dish image; however, the embodiment is not limited to this. The counting unit 112 may also calculate an image feature value as follows. For example, the counting unit 112 irradiates each of the coordinate positions of the petri dish image with light having a plurality of wavelengths and performs Fourier transformation on the transmitted light. Then, the counting unit 112 creates a matrix based on the light level of the transmitted light for each wavelength of each of the coordinates. Then, the calculating unit 113 calculates the similarity between the petri dish images based on the created matrix.

In the first embodiment, it has been described that the counting unit 112 calculates one of the color histogram, the histogram of the colony area, the average value of the area of a colonies, and the SIFT feature value as an image feature value of a petri dish image; however, the embodiment is not limited to this. The colony inspection device 100 may also evaluate the inspection quality as follows. For example, the counting unit 112 calculates a color histogram, a histogram of the colony area, the average value of the area of the colonies, and the SIFT feature value as an image feature value. Then, the calculating unit 113 calculates each of the similarities of the color histogram, the histogram of the colony area, the average value of the areas of the colonies, and the SIFT feature value. Then, by judging whether each of the similarities is equal to or greater than a threshold that are set individually, the judgement unit 114 judges whether a petri dish that is the inspection target was imperfect in the process of the inspection.

Furthermore, it has been described that the inspection department in the food manufacturing plant uses the colony inspection device 100 in order to improve the inspection quality; however, a quality management department in another department may also use the colony inspection device 100 in order to audit the inspection quality in the inspection department. Furthermore, a public institution, an external third party institution may also audit the inspection performed in the inspection department by using the colony inspection device 100.

(Update of the threshold with respect to the similarity)

The judgement unit 114 judges whether the petri dish that is the inspection target was imperfect in the process of the inspection based on whether the similarity is equal to or greater than the threshold. The threshold is set for each food group and inspection item. The threshold may also be appropriately updated by the responsible person.

For example, the colony inspection device 100 may also update the threshold in the following case. The displaying unit 102 outputs an alarm when the similarity is equal to or less than the threshold. At this point, if "within the prescription" is selected as the judgement result by the responsible person, the colony inspection device 100 may also update the threshold to a value lower than the current value. In this way, the colony inspection device 100 reflects the threshold data 123 by feeding back the judgement result obtained by the responsible person and allows the threshold to be learned based on the judgement result, whereby it is possible to set a further appropriate threshold.

(Confusion prevention of the same image)

In the first embodiment, if the similarity between the petri dish images is equal to or lower than a first threshold, the judgement unit 114 judges that the petri dish that is the inspection target was imperfect in the process of the inspection. Furthermore, if the similarity between the petri dish images is equal to or greater than a second threshold that is greater than the first threshold, the judgement unit 114 may also judge that the petri dish images are the same. Consequently, the colony inspection device 100 can prevent the same petri dish images from being confusedly displayed on the judgement screen 400 due to misconception by the responsible person. Furthermore, if the similarity between the petri dish images is equal to or greater than the second threshold, the colony inspection device 100 may also output the alarm with the content indicating that "the displayed petri dish images may possibly the same". Consequently, it is possible to prevent a fraud of evaluating inspection quality by using the same petri dish images.

For example, it is assumed that the judgement unit 114 outputs an alarm if the numbers of counts differ by "7" or more or if the similarity is equal to or less than "0.6" or is equal to or greater than "0.95". In this case, for the inspection No "107" in the inspection DB 121 illustrated in FIG. 3, because the similarity is "0.99" and thus the similarity is equal to or greater than "0.95", the judgement unit 114 judges that the condition of the alarm is satisfied and then outputs the alarm to the judgement screen 400.

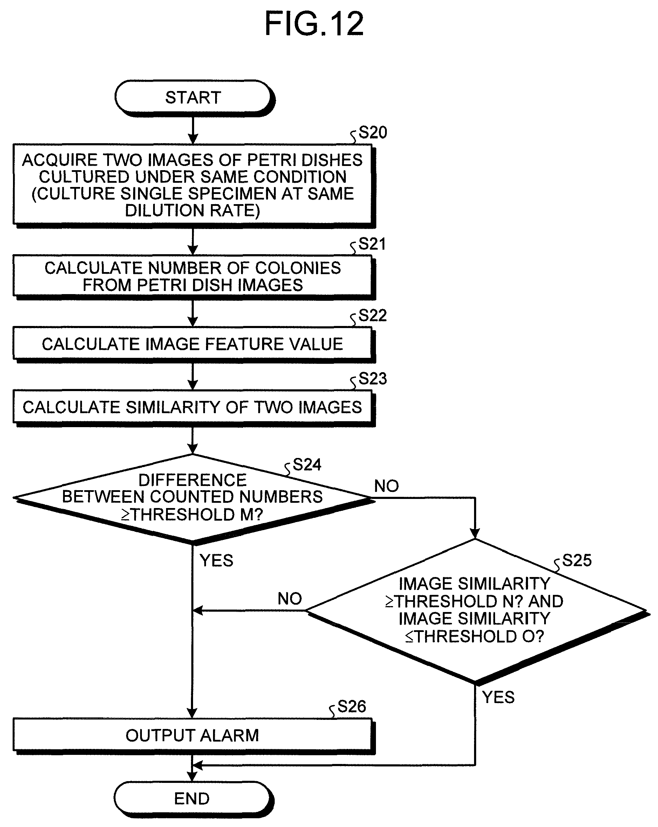

In the following, a description will be given of the flow of a process performed by the colony inspection device 100 when an alarm is output in order to prevent the confusion of the same images. FIG. 12 is a schematic diagram illustrating a second example of the processing operation performed until the colony inspection device outputs the alarm.

First, the acquiring unit 111 acquires each of the images related to the two petri dishes obtained from a single specimen that is divided and cultured at the same dilution rate (Step S20). Then, the counting unit 112 calculates each of the numbers of colonies of each of the associated petri dish images stored in the inspection DB 121 (Step S21). Then, the counting unit 112 calculates each of the image feature values from the two associated petri dish images (Step S22). Then, the calculating unit 113 calculates the similarity between the two petri dish images (Step S23).

Then, if the difference of the number of colonies between the petri dishes counted by the counting unit 112 is equal to or greater than the threshold M (Yes at Step S24), the judgement unit 114 outputs the alarm (Step S26) and ends the process. In contrast, if the difference of the number of colonies between the petri dishes counted by the counting unit 112 is smaller than the threshold M (No at Step S24), the judgement unit 114 moves to the process at Step S25. Then, if the image similarity is smaller than the threshold N or greater than the threshold O at Step S25 (No at Step S25), the judgement unit 114 outputs the alarm (Step S26) and ends the process. In contrast, if the image similarity is equal to or greater than the threshold N and is equal to or less than the threshold O at Step S25 (Yes at Step S25), the judgement unit 114 does not output the alarm and ends the process. Furthermore, the threshold N corresponds to the first threshold. Furthermore, the threshold O corresponds to the second threshold.

(Total area of the colonies in the petri dish)

If the total area of the colonies in a petri dish becomes more than half of the petri dish for the reason that a single colony highly grows, in some cases, the counting unit 112 is not able to accurately count the number of colonies. Thus, if the total area of the colonies in the petri dish calculated by the calculating unit 113 is equal to or greater than the threshold, the judgement unit 114 may also output an alarm in order to urge the responsible person to perform an additional inspection. Consequently, because the colony in which the degree of growth is large is overlapped with another colony, the colony inspection device 100 can prevent the accuracy of counting the number of colonies from being decreased.

The counting unit 112 calculates the total area of the colonies as follows. For example, the counting unit 112 performs an edge extraction process on the image 1 and the image 2 and detects the closed portion as a colony. Then, the counting unit 112 obtains the total area of the colonies by counting the number of pixels included in each of the detected colonies. Alternatively, the counting unit 112 may also count the number of pixels of the white color included in the petri dish image that was captured by being placed on a dark color board and may also calculate the total area of the colonies based on the ratio of the number of occupied white pixels to the number of pixels in the petri dish.

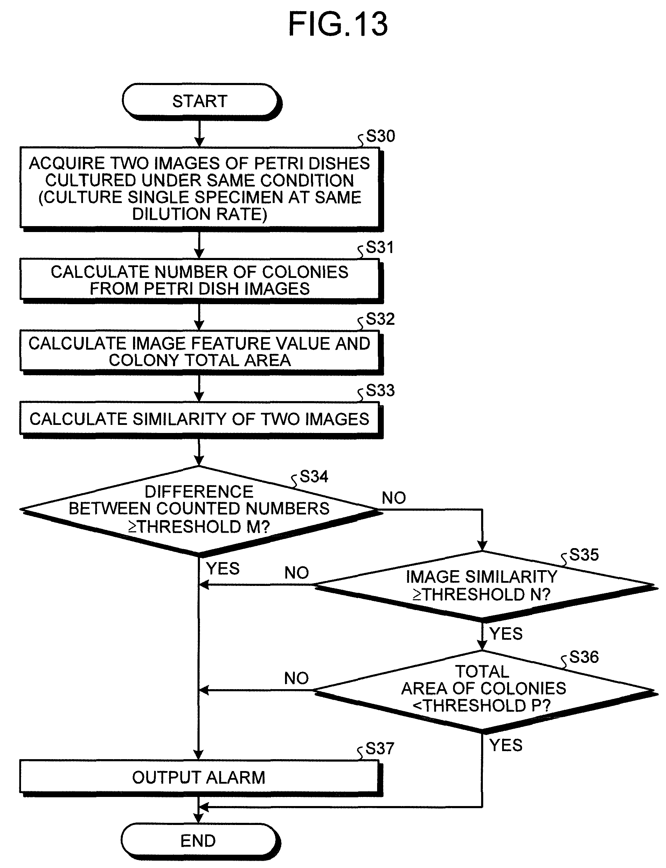

In the following, a description will be given of the flow of the process performed when the colony inspection device 100 calculates the total area of the colonies in the petri dish. FIG. 13 is a schematic diagram illustrating a third example of the processing operation performed until the colony inspection device outputs the alarm.

First, the acquiring unit 111 acquires each of the images related to the two petri dishes obtained from a single specimen that is divided and cultured at the same dilution rate (Step S30). Then, the counting unit 112 calculates each of the numbers of colonies of each of the associated petri dish images stored in the inspection DB 121 (Step S31). Then, the counting unit 112 calculates each of the image feature value and the colony total area from the associated two petri dish images (Step S32). Then, the calculating unit 113 calculates the similarity of the two petri dish images (Step S33).

Then, if the difference of the number of colonies between the petri dishes counted by the counting unit 112 is equal to or greater than the threshold M (Yes at Step S34), the judgement unit 114 outputs the alarm (Step S37) and ends the process. In contrast, if the difference of the number of colonies between the petri dishes counted by the counting unit 112 is smaller than the threshold M (No at Step S34), the judgement unit 114 moves to the process at Step S35. Then, if the image similarity is smaller than the threshold N at Step S35 (No at Step S35), the judgement unit 114 outputs the alarm (Step S37) and ends the process. In contrast, if the image similarity is equal to or greater than the threshold N at Step S35 (Yes at Step S35), the judgement unit 114 moves to the process at Step S36. Then, if the total area of the colonies is equal to or greater than a threshold P (No at Step S36), the judgement unit 114 outputs the alarm (Step S37) and ends the process. In contrast, if the total area of the colonies is smaller than a threshold P (Yes at Step S36), the judgement unit 114 does not output the alarm and ends the process.

# EP 3 050 958 A1

(Hardware configuration of the display terminal)

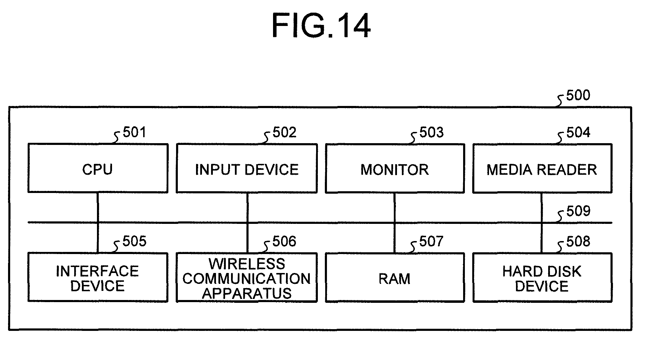

FIG. 14 is a block diagram illustrating an example of the hardware configuration of a computer according to the colony inspection device. As illustrated FIG. 14, a computer 500 includes a CPU 501 that executes various kinds of arithmetic processing, an input device 502 that receives an input of data from a user, and a monitor 503. Furthermore, the computer 500 includes a media reader 504 that reads a program or the like from a storage medium, an interface device 505 that is used to connect another device, and a wireless communication apparatus 506 that is used to wirelessly connect to the other device. Furthermore, the computer 500 includes a random access memory (RAM) 507 that temporarily stores therein various kinds of information and a hard disk device 508. Furthermore, each of the devices 501 to 508 is connected to a bus 509.

The hard disk device 508 stores therein a colony inspection program having the same function as that performed by each of the processing units, such as the acquiring unit 111, the counting unit 112, the calculating unit 113, and the judgement unit 114 in the control unit 110 illustrated in FIG. 2. Furthermore, the hard disk device 508 stores therein various kinds of data that implements the colony inspection program.

The CPU 501 reads each of the programs stored in the hard disk device 508 and loads the programs in the RAM 507, thereby performing various kinds of processes. Furthermore, these programs allow the computer 500 to function as the acquiring unit 111, the counting unit 112, the calculating unit 113, and the judgement unit 114 illustrated in FIG. 2.

Furthermore, the colony inspection program described above is not always stored in the hard disk device 508. For example, the computer 500 may also read and execute the program stored in a storage medium from which the computer 500 can read. Examples of the storage medium from which the computer 500 can read include, for example, a portable recording medium, such as a CD-ROM, a DVD disk, a universal serial bus (USB) memory, or the like; a semiconductor memory, such as a flash memory, or the like; a hard disk drive; and the like. Furthermore, the program may also be stored in a device connected to a public circuit, the Internet, local area network (LAN), or the like and the computer 500 may also read and execute the program from the storage medium described above.

[Explanation of Reference]

100 colony inspection device

101 I/F
102 displaying unit
110 control unit
111 acquiring unit
112 counting unit
113 calculating unit
114 judgement unit
120 storing unit
121 inspection DB
122 image data
123 image feature value data
124 threshold data

## Claims

1. A colony inspection program that causes a computer to execute a process comprising:

    acquiring each of captured images of a plurality of petri dishes in each of which bacterial colonies are included;
    calculating similarity between the plurality of the images; and
    controlling, based on the calculated similarity, whether an alarm is output.

2. The colony inspection program according to claim 1, wherein the changing whether the alarm is output includes outputting the alarm when the calculated similarity indicates a difference equal to or greater than a predetermined value.

3. The colony inspection program according to claim 1, wherein
    the process further comprises counting a number of the bacterial colonies included in each of the plurality of the images, and

the changing whether the alarm is output includes outputting the alarm when a difference between values counted for each of the plurality of the images is equal to or greater than a predetermined number of the bacterial colonies and when the similarity indicates a difference equal to or greater than a predetermined value.

4. A colony inspection device comprising:

an acquiring unit configured to acquire each of captured images of a plurality of petri dishes in each of which bacterial colonies are included;
a calculating unit configured to calculate the similarity between the plurality of the images; and
an output unit configured to change, based on the calculated similarity, whether an alarm is output.

5. The colony inspection device according to claim 4, wherein, the output unit is configured to output the alarm when the calculated similarity indicates a difference equal to or greater than a predetermined value.

6. The colony inspection device according to claim 4, further comprising a counting unit configured to count a number of the bacterial colonies included in each of the plurality of the images, wherein
the output unit is configured to output the alarm when a difference between values counted for each of the plurality of the images is equal to or greater than a predetermined value and when the similarity indicates a difference equal to or greater than the predetermined value.

7. A colony inspection method comprising:

acquiring, performed by a computer, each of captured images of a plurality of petri dishes in each of which bacterial colonies are included;
calculating, performed by the computer, the similarity between the plurality of the images; and
changing, performed by the computer, based on the calculated similarity, whether an alarm is output.

8. The colony inspection method according to claim 7, wherein the changing whether the alarm is output includes outputting the alarm when the calculated similarity indicates a difference equal to or greater than a predetermined value.

9. The colony inspection method according to claim 7, further comprising, performed by the computer, counting a number of the bacterial colonies included in each of the plurality of the images, wherein
the changing whether the alarm is output includes outputting the alarm when a difference between values counted for each of the plurality of the images is equal to or greater than a predetermined value and when the similarity indicates a difference equal to or greater than the predetermined value.

# FIG.1

```
┌─────────────────┐     ┌──────────┐
│ ⌇200a           │     │ ⌇300a    │                    ⌇10
│ IMAGING DEVICE  │─────│ TERMINAL │
│                 │     │ DEVICE   │
└─────────────────┘     └──────────┘

┌─────────────────┐     ┌──────────┐      ⌇50      ┌──────────┐
│ ⌇200b           │     │ ⌇300b    │               │ ⌇100     │
│ IMAGING DEVICE  │─────│ TERMINAL │               │ COLONY   │
│                 │     │ DEVICE   │               │ INSPEC-  │
└─────────────────┘     └──────────┘               │ TION     │
                                                   │ DEVICE   │
┌─────────────────┐     ┌──────────┐               │          │
│ ⌇200c           │     │ ⌇300c    │               │          │
│ IMAGING DEVICE  │─────│ TERMINAL │               └──────────┘
│                 │     │ DEVICE   │
└─────────────────┘     └──────────┘
```

# FIG.2

```
                                                    ⌇100
┌──────────────────────────────────────────────────────────────┐
│                  COLONY INSPECTION DEVICE                      │
│                                                                │
│                      ⌇110              ⌇120                    │
│              ┌──────────────┐   ┌──────────────┐               │
│              │ CONTROL UNIT │   │ STORING UNIT │               │
│              │    ⌇111      │   │    ⌇121      │               │
│              │ ┌──────────┐ │   │ ┌──────────┐ │               │
│              │ │ ACQUIRING│ │   │ │INSPECTION│ │               │
│              │ │ UNIT     │ │   │ │ DB       │ │               │
│  ⌇101        │ └──────────┘ │   │ └──────────┘ │               │
│ ┌──────┐     │    ⌇112      │   │    ⌇122      │               │
│ │ I/F  │─────│ ┌──────────┐ │   │ ┌──────────┐ │               │
│ └──────┘     │ │ COUNTING │ │   │ │IMAGE DATA│ │               │
│              │ │ UNIT     │ │   │ └──────────┘ │               │
│  ⌇102        │ └──────────┘ │   │    ⌇123      │               │
│ ┌──────────┐ │    ⌇113      │   │ ┌──────────┐ │               │
│ │DISPLAYING│ │ ┌──────────┐ │   │ │ IMAGE    │ │               │
│ │UNIT      │ │ │CALCULATING││   │ │ FEATURE  │ │               │
│ └──────────┘ │ │UNIT      │ │   │ │VALUE DATA│ │               │
│              │ └──────────┘ │   │ └──────────┘ │               │
│              │    ⌇114      │   │    ⌇124      │               │
│              │ ┌──────────┐ │   │ ┌──────────┐ │               │
│              │ │ JUDGEMENT│ │   │ │ THRESHOLD│ │               │
│              │ │ UNIT     │ │   │ │ DATA     │ │               │
│              │ └──────────┘ │   │ └──────────┘ │               │
│              └──────────────┘   └──────────────┘               │
└──────────────────────────────────────────────────────────────┘
```

# FIG.3

| INSPEC-TION NO | SPECI-MEN 1 | SPECI-MEN 2 | INSPEC-TION DATE | SPECIMEN NAME | cfu | DILUTION RATE | NUMBER OF COUNTS 1 | NUMBER OF COUNTS 2 | SIMI-LARITY | JUDGE-MENT RESULT |
|---|---|---|---|---|---|---|---|---|---|---|
| 105 | IMAGE 1 | IMAGE 2 | 20xx/xx/xx | CUT CABBAGE | $1.8 \times 10^3$ | $\times 10^2$ | 19 | 17 | 0.55 | L.A. |
| 106 | IMAGE 3 | IMAGE 4 | 20xx/xx/xx | CUT CABBAGE | $2.2 \times 10^3$ | $\times 10^2$ | 16 | 28 | 0.61 | L.A. |
| 107 | IMAGE 5 | IMAGE 5 | 20xx/xx/xx | CUT ONION | $1.2 \times 10^3$ | $\times 10^2$ | 12 | 12 | 0.99 | L.A. |
| 108 | IMAGE 7 | IMAGE 6 | 20xx/xx/xx | POTATO SALAD | $1.7 \times 10^3$ | $\times 10^2$ | 16 | 17 | 0.71 | WITHIN PRESCRIP-TION |
| 109 | IMAGE 9 | IMAGE 10 | 20xx/xx/xx | WIENER | $2.0 \times 10^3$ | $\times 10^2$ | 18 | 21 | 0.55 | OUTSIDE PRESCRIP-TION |

EP 3 050 958 A1

# FIG.4

# FIG.5

# FIG.6

# FIG.7

# FIG.8

# FIG.9

~400

**RESULT JUDGEMENT**

**RESPONSIBLE PERSON NAME: xx xx**

~410

402a 402b

| 1 | 2 |

~401b

INSPECTION NO. 105

INSPECTION DATE
20xx/xx/xx

SPECIMEN NAME
CUT CABBAGE

cfu $1.8 \times 10^3$ cfu/g

DILUTION RATE $\times 10^2$

NUMBER OF
COUNTS 1     19

NUMBER OF
COUNTS 2     17

~411

THERE IS POSSIBILITY
OF PROBLEM WITH
INSPECTION QUALITY

~421

| WITHIN PRESCRIP-TION |
|---|

~422

| OUTSIDE PRESCRIP-TION |
|---|

~423

| L.A. |
|---|

| CONFIR-MATION |
|---|

~430

# FIG.10

| | | DIFFERENCE BETWEEN COLONY COUNT | |
| --- | --- | --- | --- |
| | | SMALL | LARGE |
| SIMILARLITY OF PETRI DISHES | HIGH (SIMILAR) | NO PROBLEM | THERE IS HIGH POSSIBILITY OF IMPERFECTION IN ONE OF PETRI DISHES IN PROCESS OF INSPECTION ↓ OUTPUT ALARM IN ACCORDANCE WITH DIFFERENCE BETWEEN NUMBER OF COUNTS |
| | LOW (NOT SIMILAR) | NUMBER OF COUNTS DO NOT DIFFER BUT DEVELOPMENT SITUATIONS OF NUMBER OF COLONIES DIFFER ↓ OUTPUT ALARM IN ACCORDANCE WITH DIFFERENCE BETWEEN SIMILARLITY OF IMAGES | |

# FIG.11

```
                      ┌──────────────┐
                      │    START     │
                      └──────┬───────┘
                             │            ⌐S10
    ┌────────────────────────▼────────────────────────┐
    │   ACQUIRE TWO IMAGES OF PETRI DISHES             │
    │   CULTURED UNDER SAME CONDITION                  │
    │   (CULTURE SINGLE SPECIMEN AT SAME               │
    │           DILUTION RATE)                         │
    └────────────────────────┬────────────────────────┘
                             │            ⌐S11
    ┌────────────────────────▼────────────────────────┐
    │   CALCULATE NUMBER OF COLONIES                   │
    │        FROM PETRI DISH IMAGES                    │
    └────────────────────────┬────────────────────────┘
                             │            ⌐S12
    ┌────────────────────────▼────────────────────────┐
    │      CALCULATE IMAGE FEATURE VALUE               │
    └────────────────────────┬────────────────────────┘
                             │            ⌐S13
    ┌────────────────────────▼────────────────────────┐
    │    CALCULATE SIMILARITY OF TWO IMAGES            │
    └────────────────────────┬────────────────────────┘
                             │            ⌐S14
```

DIFFERENCE BETWEEN COUNTED NUMBERS ≥THRESHOLD M?

NO

YES     NO

S15

IMAGE SIMILARITY ≥THRESHOLD N?

YES

S16

OUTPUT ALARM

END

# FIG.12

```
                    ( START )
                        |
                        v
┌───────────────────────────────────────────┐  ⌐S20
│  ACQUIRE TWO IMAGES OF PETRI DISHES         │
│  CULTURED UNDER SAME CONDITION              │
│  (CULTURE SINGLE SPECIMEN AT SAME           │
│  DILUTION RATE)                             │
└───────────────────────────────────────────┘
                        |
                        v
┌───────────────────────────────────────────┐  ⌐S21
│  CALCULATE NUMBER OF COLONIES               │
│  FROM PETRI DISH IMAGES                     │
└───────────────────────────────────────────┘
                        |
                        v
┌───────────────────────────────────────────┐  ⌐S22
│  CALCULATE IMAGE FEATURE VALUE              │
└───────────────────────────────────────────┘
                        |
                        v
┌───────────────────────────────────────────┐  ⌐S23
│  CALCULATE SIMILARITY OF TWO IMAGES         │
└───────────────────────────────────────────┘
                        |
                        v
```

S24
DIFFERENCE
BETWEEN COUNTED NUMBERS
≥THRESHOLD M?

NO

YES

S25
IMAGE SIMILARITY
≥THRESHOLD N? AND
IMAGE SIMILARITY
≤THRESHOLD O?

NO

YES

```
┌───────────────────────────────────────────┐  ⌐S26
│  OUTPUT ALARM                               │
└───────────────────────────────────────────┘
                        |
                        v
                    (  END  )
```

# FIG.13

START

↓

**S30**

ACQUIRE TWO IMAGES OF PETRI DISHES
CULTURED UNDER SAME CONDITION
(CULTURE SINGLE SPECIMEN AT SAME
DILUTION RATE)

↓

**S31**

CALCULATE NUMBER OF COLONIES
FROM PETRI DISH IMAGES

↓

**S32**

CALCULATE IMAGE FEATURE VALUE AND
COLONY TOTAL AREA

↓

**S33**

CALCULATE SIMILARITY OF TWO IMAGES

↓

**S34**
DIFFERENCE
BETWEEN COUNTED NUMBERS
≥THRESHOLD M? —— NO →

**S35**
IMAGE SIMILARITY
≥THRESHOLD N?

← NO

YES ↓

**S36**
TOTAL
AREA OF COLONIES
<THRESHOLD P?

← NO

YES ↓

YES ↓

**S37**

OUTPUT ALARM

↓

END

# FIG.14

```
                                                                    ⌐500
┌─────────────────────────────────────────────────────────────────────────┐
│                                                                          │
│        ⌐501              ⌐502              ⌐503              ⌐504         │
│  ┌──────────────┐  ┌──────────────┐  ┌──────────────┐  ┌──────────────┐  │
│  │              │  │              │  │              │  │              │  │
│  │     CPU      │  │ INPUT DEVICE │  │   MONITOR    │  │ MEDIA READER │  │
│  │              │  │              │  │              │  │              │  │
│  └──────────────┘  └──────────────┘  └──────────────┘  └──────────────┘  │
│                                                                  ⌐509    │
│                                                                          │
│        ⌐505              ⌐506              ⌐507              ⌐508         │
│  ┌──────────────┐  ┌──────────────┐  ┌──────────────┐  ┌──────────────┐  │
│  │              │  │  WIRELESS    │  │              │  │              │  │
│  │  INTERFACE   │  │COMMUNICATION │  │     RAM      │  │  HARD DISK   │  │
│  │   DEVICE     │  │  APPARATUS   │  │              │  │   DEVICE     │  │
│  └──────────────┘  └──────────────┘  └──────────────┘  └──────────────┘  │
│                                                                          │
└─────────────────────────────────────────────────────────────────────────┘
```

## INTERNATIONAL SEARCH REPORT

International application No.

PCT/JP2013/075778

### A. CLASSIFICATION OF SUBJECT MATTER
*C12M1/34*(2006.01)i, *G01N21/17*(2006.01)i

According to International Patent Classification (IPC) or to both national classification and IPC

### B. FIELDS SEARCHED

Minimum documentation searched (classification system followed by classification symbols)
C12M1/34, G01N21/17

Documentation searched other than minimum documentation to the extent that such documents are included in the fields searched

| | | | |
|---|---|---|---|
| Jitsuyo Shinan Koho | 1922–1996 | Jitsuyo Shinan Toroku Koho | 1996–2013 |
| Kokai Jitsuyo Shinan Koho | 1971–2013 | Toroku Jitsuyo Shinan Koho | 1994–2013 |

Electronic data base consulted during the international search (name of data base and, where practicable, search terms used)
JSTPlus/JMEDPlus/JST7580(JDreamIII)

### C. DOCUMENTS CONSIDERED TO BE RELEVANT

| Category* | Citation of document, with indication, where appropriate, of the relevant passages | Relevant to claim No. |
|---|---|---|
| X | JP 2012-135240 A (Hitachi High-Technologies Corp.), 19 July 2012 (19.07.2012), claims 1, 6; paragraphs [0017] to [0018], [0029], [0034], [0055] (Family: none) | 1-9 |
| A | JP 2011-239683 A (Hitachi High-Technologies Corp.), 01 December 2011 (01.12.2011), paragraphs [0028] to [0037] (Family: none) | 1-9 |
| A | JP 2011-212013 A (Elmex Ltd.), 27 October 2011 (27.10.2011), paragraphs [0002] to [0022] & WO 2011/115218 A1 | 1-9 |

☐ Further documents are listed in the continuation of Box C.  ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 18 October, 2013 (18.10.13) | 29 October, 2013 (29.10.13) |

| Name and mailing address of the ISA/ Japanese Patent Office | Authorized officer |
|---|---|
| Facsimile No. | Telephone No. |

Form PCT/ISA/210 (second sheet) (July 2009)

**EP 3 050 958 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011239683 A
- JP 2011212013 A